# EUROPEAN PATENT APPLICATION

(11) **EP 4 523 679 A1**
(43) Date of publication of application: **19.03.2025**
(21) Application number: 24197366.8
(22) Date of filing: 29.08.2024
(51) Int. Cl.: A61K 9/00, A61K 9/08, A61K 47/10, A61K 47/26, A61K 47/32, A61K 47/38

(54) **EYE DROP FORMULATIONS IN THE FORM OF AN AQUEOUS SOLUTION COMPRISING 8-OXO-2'-DEOXYGUANOSINE OR A PHARMACEUTICALLY ACCEPTABLE SALT THEREOF**

(30) Priority: 13.09.2023 KR 20230121513
(71) Applicant: Rudacure Corporation, Incheon 21988 (KR)
(72) Inventor: KIM, Dong Hyun, 18488 Hwaseong-si (KR); PARK, Seol Gyu, 17046 Yongin-si (KR); LEE, Dong Jin, 18238 Hwaseong-si (KR); KIM, Sun Kyung, 16996 Yongin-si (KR)
(74) Representative: Turner, Craig Robert

(57) **Abstract**

The present invention provides an eye drop formulation in the form of an aqueous solution, comprising 8-oxo-2'-deoxyguanosine or a pharmaceutically acceptable salt thereof; a thickening agent; a buffering agent; and a chelating agent, in an aqueous medium, wherein the eye drop formulation has a viscosity of 10 to 30 mPa·s. The eye drop formulation of the present invention exhibits suitable retention time in the eye as well as excellent stability without precipitation even when stored for a long period of time.

## Description

### TECHNICAL FIELD

The present invention relates to an eye drop formulation in the form of an aqueous solution comprising 8-oxo-2'-deoxyguanosine or a pharmaceutically acceptable salt thereof. More specifically, the present invention relates to an eye drop formulation in the form of an aqueous solution comprising 8-oxo-2'-deoxyguanosine or a pharmaceutically acceptable salt thereof as an active ingredient and having a certain viscosity.

### BACKGROUND ART

The compound of Formula 1, whose chemical name is 8-oxo-2'-deoxyguanosine, or a pharmaceutically acceptable salt thereof exhibits rapid corneal epithelial restoration and therefore is useful for preventing and/or treating corneal injuries, including dry eye syndrome, eye trauma, and infectious or non-infectious uveitis (Korean Patent No. 10-1816277 and US Patent No. 10,675,294).

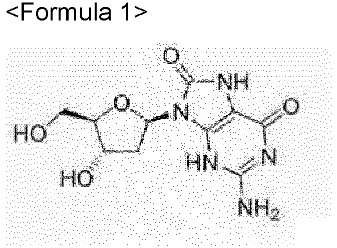

In order to achieve effective prevention and/or treatment of corneal injuries through repeated instillations, a stable eye drop formulation in the form of an aqueous solution comprising a therapeutically effective amount of the compound of Formula 1 or a pharmaceutically acceptable salt thereof is required.

However, since the compound of Formula 1 has a very low water solubility of 1.91 mg/mL at room temperature (about 25°C), it is difficult to prepare an eye drop formulation in the form of an aqueous solution comprising a therapeutically effective amount thereof.

It may be tried to solubilize the compound of Formula 1 by heating a mixture containing the compound of Formula 1 in aqueous solution (e.g., a mixture in the form of a suspension). However, when the aqueous solution obtained by heating is stored at room temperature, the compound of Formula 1 precipitates, making it difficult to be used as an eye drop formulation that requires repeated instillations.

Additionally, it may also be tried to solubilize the compound of Formula 1 by increasing the pH of a mixture containing the compound of Formula 1 in aqueous solution (e.g., a mixture in the form of a suspension). However, there is a problem that the compound of Formula 1 cannot be solubilized in the pH range that can be used as an eye drop formulation (i.e., in pH 5 to 7).

In addition, when a patient repeatedly uses an eye drop formulation in the form of an aqueous solution, a significant amount of the drug solution leaks from the eye depending on the method of use. Said leakage often causes the problem that desired therapeutically effective amounts of the eye drop formulation cannot be retained in the eye. Therefore, it is required to prepare an eye drop formulation in the form of an aqueous solution which exhibits a suitable retention time in the eye.

### DISCLOSURE

### Technical Problem

The present inventors carried out various researches in order to develop an eye drop formulation in the form of a solution comprising the compound of Formula 1 or a pharmaceutically acceptable salt thereof in an aqueous medium, which can solve the above-mentioned problems. As the results thereof, the present inventors have found that an eye drop formulation in the form of an aqueous solution having a certain viscosity (i.e., a viscosity of 10 to 30 mPa s) and solubilizing the compound of formula 1 or a pharmaceutically acceptable salt thereof exhibits a suitable retention time in the eye. In addition, the present inventors have found that a combination of certain ingredients (thickening agents) not only functions as a thickening agent, but also functions as a stabilizer and solubilizer, thereby providing an eye drop formulation in the form of an aqueous solution having excellent stability. Especially, the present inventors have found that, when an eye drop formulation in the form of an aqueous solution is prepared by using a certain solubilizing agent in addition to the combination of the above-described thickening agents, a stable eye drop that does not show precipitation under a room temperature condition for 12 months can be obtained.

Therefore, it is an object of the present invention to provide an eye drop formulation in the form of a solution, comprising 8-oxo-2'-deoxyguanosine or a pharmaceutically acceptable salt thereof as an active ingredient, wherein the eye drop formulation has a certain viscosity.

### Technical Solution

In accordance with an aspect of the present invention, there is provided an eye drop formulation in the form of an aqueous solution, comprising 8-oxo-2'-deoxyguanosine or a pharmaceutically acceptable salt thereof; a thickening agent; a buffering agent; and a chelating agent, in an aqueous medium, wherein the eye drop formulation has a viscosity of 10 to 30 mPa·s.

In an embodiment, the eye drop formulation of the present invention does not show precipitation when stored under a refrigerated condition of about 4°C for 30 days. In another embodiment, the eye drop formulation of the present invention does not show precipitation when stored under a room temperature of about 25°C for 60 days.

In the eye drop formulation of the present invention, 8-oxo-2'-deoxyguanosine or a pharmaceutically acceptable salt thereof may be present in a concentration of 0.1 to 1.0 w/v%. In the eye drop formulation of the present invention, the buffering agent may be tromethamine, borax, boric acid, potassium dihydrogen phosphate, potassium monohydrogen phosphate, sodium chloride, sodium hydroxide, sodium carbonate, or potassium carbonate; and the chelating agent may be ethylenediaminetetraacetic acid or a salt thereof, citric acid or a salt thereof, metaphosphoric acid or a salt thereof, or polyphosphoric acid or a salt thereof.

In the eye drop formulation of the present invention, the thickening agent may comprise a combination of polyvinylpyrrolidone and hydroxypropyl methylcellulose. The polyvinylpyrrolidone may have a weight average molecular weight of 20,000 to 100,000 and may be present in a concentration of 0.5 to 5.0 w/v%. The hydroxypropyl methylcellulose may have a weight average molecular weight of 10,000 to 1,500,000 and may be present in a concentration of 0.1 to 0.4 w/v%.

The eye drop formulation of the present invention may further comprise polyethylene glycol and polysorbate as a solubilizing agent. The polyethylene glycol may have a weight average molecular weight of 380 to 420 and may be present in a concentration of 2.0 to 5.0 w/v%. The polysorbate may be polysorbate 20, polysorbate 40, polysorbate 60, or polysorbate 80 and may be present in a concentration of 0.05 to 0.15 w/v%.

### ADVANTAGEOUS EFFECTS

It has been found by the present invention that the eye drop formulation in the form of an aqueous solution having a certain viscosity (i.e., a viscosity of 10 to 30 mPa s) and solubilizing the compound of formula 1 or a pharmaceutically acceptable salt thereof exhibits a suitable retention time in the eye. In addition, it has been found by the present invention that the combination of polyvinylpyrrolidone and hydroxypropyl methylcellulose not only functions as a thickening agent, but also functions as a stabilizer and solubilizer, thereby providing an eye drop formulation in the form of an aqueous solution having excellent stability. Especially, it has been also found by the present invention that, when an eye drop formulation in the form of an aqueous solution is prepared by using a certain solubilizing agent (i.e., polyethylene glycol and polysorbate) in addition to the combination of polyvinylpyrrolidone and hydroxypropyl methylcellulose, a stable eye drop that does not show precipitation under a room temperature condition for 12 months can be obtained. Therefore, the eye drop formulation of the present invention exhibits suitable retention time in the eye as well as excellent stability without precipitation even when stored for a long period of time, thereby being able to be usefully applied for long-term and repeated administrations (repeated instillations).

### BEST MODE

The present invention provides an eye drop formulation in the form of an aqueous solution, comprising 8-oxo-2'-deoxyguanosine or a pharmaceutically acceptable salt thereof; a thickening agent; a buffering agent; and a chelating agent, in an aqueous medium, wherein the eye drop formulation has a viscosity of 10 to 30 mPa·s.

In an embodiment, the eye drop formulation of the present invention does not show precipitation when stored under a refrigerated condition of about 4°C for 30 days. In another embodiment, the eye drop formulation of the present invention does not show precipitation occurs when stored under a room temperature of about 25°C for 60 days.

In the eye drop formulation of the present invention, the aqueous medium includes distilled water for injection, sterilized purified water, physiological saline, etc.

In the eye drop formulation of the present invention, 8-oxo-2'-deoxyguanosine or a pharmaceutically acceptable salt thereof may be used in a therapeutically effective amount. For example, the eye drop formulation of the present invention may comprise 8-oxo-2'-deoxyguanosine or a pharmaceutically acceptable salt thereof in a concentration of 0.1 to 1.0 w/v%, preferably about 0.25 w/v%, but not limited thereto. The pharmaceutically acceptable salt of 8-oxo-2'-deoxyguanosine may be selected from the various salts disclosed in Korean Patent No. 10-1816277, for example, the acid addition salts thereof.

The eye drop formulation of the present invention may comprise an excipient such as a buffering agent, a chelating agent, etc. The buffering agent may be tromethamine, borax, boric acid, potassium dihydrogen phosphate, potassium monohydrogen phosphate, sodium chloride, sodium hydroxide, sodium carbonate, or potassium carbonate, preferably tromethamine. The buffering agent may be used in an amount sufficient for providing an appropriate buffering effect. For example, the buffering agent may be present in a concentration of 0.5 to 2.0 w/v%, preferably about 1.0 w/v%. The chelating agent may be ethylenediaminetetraacetic acid or a salt thereof, citric acid or a salt thereof, metaphosphoric acid or a salt thereof, or polyphosphoric acid or a salt thereof, preferably sodium ethylenediaminetetraacetate. The chelating agent may be used in an amount conventionally used in the field of eye drop formulations. For example, the chelating agent may be present in a concentration of 0.01 to 0.3 w/v%, preferably about 0.1 w/v%.

It has been found by the present invention that a certain thickening agent, i.e., the combination of polyvinylpyrrolidone and hydroxypropyl methylcellulose, not only functions as a thickening agent, but also functions as a stabilizer and solubilizer, thereby providing an eye drop formulation in the form of an aqueous solution having excellent stability. Therefore, the eye drop formulation of the present invention may preferably comprise a combination of polyvinylpyrrolidone and hydroxypropyl methylcellulose as a thickening agent.

The polyvinylpyrrolidone may have a weight average molecular weight of 20,000 to 100,000. Preferably, the polyvinylpyrrolidone may have a weight average molecular weight of 30,000 to 50,000 (e.g., polyvinylpyrrolidone K-25, K-30, etc.), and more preferably a weight average molecular weight of about 50,000. The polyvinylpyrrolidone may be present in a concentration of 0.5 to 5.0 w/v%, preferably 1.0 to 3.0 w/v%, more preferably about 2.0 w/v%.

The hydroxypropyl methylcellulose may have a weight average molecular weight of 10,000 to 1,500,000. Preferably, the hydroxypropyl methylcellulose may have a weight average molecular weight of 15,000 to 400,000 (e.g., hydroxypropyl methylcellulose 2910 606, hydroxypropyl methylcellulose 2910 60SH, hydroxypropyl methylcellulose 2910 E4M, hydroxypropyl methylcellulose 2910 603, etc.), more preferably a weight average molecular weight of about 400,000 (e.g., hydroxypropyl methylcellulose 2910 E4M, etc.). The hydroxypropyl methylcellulose may be present in a concentration of 0.1 to 0.4 w/v%, preferably 0.3 to 0.4 w/v%, more preferably about 0.4 w/v%.

It has been found by the present invention that, when an eye drop formulation in the form of an aqueous solution is prepared by using a certain solubilizing agent (i.e., polyethylene glycol and polysorbate) in addition to the combination of polyvinylpyrrolidone and hydroxypropyl methylcellulose, a stable eye drop that does not show precipitation under a room temperature condition for 12 months can be obtained. Therefore, the eye drop formulation of the present invention preferably further comprises polyethylene glycol and polysorbate as a solubilizing agent.

The polyethylene glycol may have a weight average molecular weight of 380 to 420. For example, polyethylene glycol 400 may be preferably used. The polyethylene glycol may be present in a concentration of 2.0 to 5.0 w/v%, preferably 2.0 to 4.0 w/v%, more preferably about 2.0 w/v%. The polysorbate may be polysorbate 20, polysorbate 40, polysorbate 60, or polysorbate 80. The polysorbate may be present at a concentration of 0.05 to 0.15 w/v%, preferably 0.1 to 0.15 w/v%, more preferably about 0.15 w/v%.

In an embodiment of the present invention, there is provided an eye drop formulation, which comprises 0.1 to 1.0 w/v% of 8-oxo-2'-deoxyguanosine or a pharmaceutically acceptable salt thereof; 0.5 to 2.0 w/v% of a buffering agent; 0.01 to 0.3 w/v% of a chelating agent; 0.5 to 5.0 w/v% of polyvinylpyrrolidone; 0.1 to 0.4 w/v% of hydroxypropyl methylcellulose; 2.0 to 5.0 w/v% of polyethylene glycol; and 0.05 to 0.15 w/v% of polysorbate, in an aqueous medium.

In a preferred embodiment of the invention, there is provided an eye drop formulation, which comprises 0.25 w/v% of 8-oxo-2'-deoxyguanosine; 1.0 w/v% of tromethamine; 0.1 w/v% of sodium ethylenediaminetetraacetate; 2.0 w/v% of polyvinylpyrrolidone; 0.4 w/v% of hydroxypropyl methylcellulose; 2.0 w/v% of polyethylene glycol; and 0.15 w/v% of polysorbate, in an aqueous medium.

The eye drop formulation in the form of an aqueous solution of the present invention may have a pH of 5.0 to 7.0, preferably about 6.5.

The present invention will be described in further detail with reference to the following examples. These examples are for illustrative purposes only and are not intended to limit the scope of the present invention.

In the following examples, HL262 means 8-oxo-2'-deoxyguanosine.

### Example 1

According to the components and amounts shown in Table 1, tromethamine, sodium edetate, and the thickening agent (polyvinylpyrrolidone and/or hydroxypropyl methylcellulose) were dissolved in sterilized purified water and then HL262 was dissolved therein. Polyvinylpyrrolidone and hydroxypropyl methylcellulose were used within the maximum amounts recommended by the FDA (HPMC: 0.5 w/v%, PVP K30: 2.0 w/v%). The pH of each resulting solution was adjusted to about pH 6.5 with a pH controlling agent (hydrochloric acid) and the final volume thereof was adjusted to about 100 mL with sterilized purified water to prepare each eye drop formulation. The viscosity of each resulting formulation was evaluated with the capillary viscometer method, the Method 1 in General Tests of the Korean Pharmacopoeia (K = 0.0839). The resulting formulations were stored at about 4°C (i.e., refrigerated condition) for 30 days and evaluated daily for precipitation. And, the resulting formulations were stored at about 25°C (i.e., room temperature condition) for 60 days and evaluated daily for precipitation. If precipitation was observed in an eye drop formulation, the precipitation evaluation thereof was discontinued. The results obtained by evaluating viscosity and precipitation as described above are shown in Table 1 below.

**Table 1**

| Component | | Examples | | | | | | |
|---|---|---|---|---|---|---|---|---|
| | | 1-1 | 1-2 | 1-3 | 1-4 | 1-5 | 1-6 | 1-7 |
| HL262 | | 0.25 g | 0.25 g | 0.25 g | 0.25 g | 0.25 g | 0.25 g | 0.25 g |
| Tromethamine | | 1.0 g | 1.0 g | 1.0 g | 1.0 g | 1.0 g | 1.0 g | 1.0 g |
| Sodium edetate | | 0.1 g | 0.1 g | 0.1 g | 0.1 g | 0.1 g | 0.1 g | 0.1 g |
| Polyvinylpyrrolidone K-30 | | 0.5 g | 2.0 g | 5.0 g | - | - | - | 2.0 g |
| Hydroxypropyl methylcellulose 2910 E4M | | - | - | - | 0.1 g | 0.2 g | 0.4 g | 0.4 g |
| pH controlling agent (pH 6.5) | | q.s. | q.s. | q.s. | q.s. | q.s. | q.s. | q.s. |
| Viscosity (mPa·s) | | 2.9 | 5.6 | 9.8 | 7.5 | 10.8 | 15.2 | 20.3 |
| Precipitation | Refrigerated condition | Precipitated (Day 3) | Precipitated (Day 5) | Precipitated (Day 5) | Precipitated (Day 4) | Precipitated (Day 5) | Precipitated (Day 7) | Not precipitat ed (Day 30) |
| | Room temperature condition | Precipitat ed (Day 7) | Precipitat ed (Day 10) | Precipitat ed (Day 10) | Precipitat ed (Day 12) | Precipitat ed (Day 12) | Precipitat ed (Day 14) | Not precipitat ed (Day 60) |

As can be seen from the results of Table 1, the use of hydroxypropyl methylcellulose in a high amount as a thickening agent or the use of the combination of polyvinylpyrrolidone and hydroxypropyl methylcellulose as a thickening agent can provide the viscosity capable of exhibiting suitable retention time in the eye (i.e., 10 to 30 mPa.s).

In addition, the use of polyvinylpyrrolidone alone or hydroxypropyl methylcellulose alone as a thickening agent showed precipitation under both refrigerated and room temperature conditions. In contrast, the use of the combination of polyvinylpyrrolidone and hydroxypropyl methylcellulose as a thickening agent showed excellent physical stability under both refrigerated and room temperature conditions. Therefore, it can be confirmed that polyvinylpyrrolidone and hydroxypropyl methylcellulose function as a stabilizer and solubilizer in addition to as a thickening agent, in a HL262-containing eye drop formulation.

### Example 2

According to the components and amounts shown in Table 2, tromethamine, sodium edetate, the thickening agent (polyvinylpyrrolidone and/or hydroxypropyl methylcellulose), and the solubilizing agent (polyethylene glycol and polysorbate) were dissolved in sterilized purified water and then HL262 was dissolved therein. The pH of each resulting solution was adjusted to about pH 6.5 with a pH controlling agent (hydrochloric acid) and the final volume thereof was adjusted to about 100 mL with sterilized purified water to prepare each eye drop formulation. The viscosity of each resulting formulation was evaluated with the capillary viscometer method, the Method 1 in General Tests of the Korean Pharmacopoeia (K = 0.0839). The resulting formulations were stored at about 4°C (i.e., refrigerated condition) for 30 days and evaluated daily for precipitation. And, the resulting formulations were stored at about 25°C (i.e., room temperature condition) for 12 months and evaluated daily for precipitation. If precipitation was observed in an eye drop formulation, the precipitation evaluation thereof was discontinued. The results obtained by evaluating viscosity and precipitation as described above are shown in Table 2 below.

**Table 2**

| Component | | Examples | | | |
|---|---|---|---|---|---|
| | | 2-1 | 2-2 | 2-3 | 2-4 |
| H L262 | | 0.25 g | 0.25 g | 0.25 g | 0.25 g |
| Tromethamine | | 1.0 g | 1.0 g | 1.0 g | 1.0 g |
| Sodium edetate hydrate | | 0.1 g | 0.1 g | 0.1 g | 0.1 g |
| Polyethylene glycol 400 | | 2.0 g | 2.0 g | 2.0 g | 2.0 g |
| Polysorbate 80 | | 0.15 g | 0.15 g | 0.15 g | 0.15 g |
| Polyvinylpyrrolidone K-30 | | - | - | 2.0 g | 2.0 g |
| Hydroxypropyl methylcellulose 2910 E4M | | - | 0.4 g | - | 0.4 g |
| pH controlling agent (pH 6.5) | | q.s. | q.s. | q.s. | q.s. |
| Viscosity (mPa·s) | | 0 | 14.4 | 7.5 | 23.1 |
| Precipitation | Refrigerated condition | Precipitated (Day 2) | Precipitated (Day 20) | Precipitated (Day 10) | Not precipitated (Day 30) |
| | Room temperature condition | Precipitated (Day 3) | Precipitated (Day 31) | Precipitated (Day 15) | Not precipitated (12 months) |

As can be seen from the results of Table 2, the formulation of Example 2-1 (which does not contain a thickening agent) and the formulation of Example 2-3 (which contains polyvinylpyrrolidone alone as a thickening agent) did not provide the viscosity capable of exhibiting suitable retention time in the eye. Said formulations also showed precipitation under both refrigerated and room temperature conditions. And, the formulation of Example 2-2 (which comprises hydroxypropyl methylcellulose alone as a thickening agent) also showed precipitation under both refrigerated and room temperature conditions. In contrast, the use of the combination of polyvinylpyrrolidone and hydroxypropyl methylcellulose as a thickening agent and the use of polyethylene glycol and polysorbate as a solubilizing agent (the formulation of Example 2-4) provided the viscosity capable of exhibiting suitable retention time in the eye. In addition, said formulations also showed excellent physical stability under both refrigerated and room temperature conditions. Especially, it can be confirmed that the eye drop formulation of Example 2-4 showed no precipitation at all under the room temperature condition for 12 months.

## Claims

1. An eye drop formulation in the form of an aqueous solution, comprising 8-oxo-2'-deoxyguanosine or a pharmaceutically acceptable salt thereof; a thickening agent; a buffering agent; and a chelating agent, in an aqueous medium, wherein the eye drop formulation has a viscosity of 10 to 30 mPa s.

2. The eye drop formulation as claimed in claim 1, wherein no precipitation occurs when stored under a refrigerated condition of about 4°C for 30 days.

3. The eye drop formulation as claimed in claim 1, wherein no precipitation occurs when stored under a room temperature of about 25°C for 60 days.

4. The eye drop formulation as claimed in claim 1, wherein 8-oxo-2'-deoxyguanosine or a pharmaceutically acceptable salt thereof is present in a concentration of 0.1 to 1.0 w/v%.

5. The eye drop formulation as claimed in claim 1, wherein the buffering agent is tromethamine, borax, boric acid, potassium dihydrogen phosphate, potassium monohydrogen phosphate, sodium chloride, sodium hydroxide, sodium carbonate, or potassium carbonate.

6. The eye drop formulation as claimed in claim 1, wherein the chelating agent is ethylenediaminetetraacetic acid or a salt thereof, citric acid or a salt thereof, metaphosphoric acid or a salt thereof, or polyphosphoric acid or a salt thereof.

7. The eye drop formulation as claimed in any one of claims 1 to 6, wherein the thickening agent comprises a combination of polyvinylpyrrolidone and hydroxypropyl methylcellulose.

8. The eye drop formulation as claimed in claim 7, wherein the polyvinylpyrrolidone has a weight average molecular weight of 20,000 to 100,000.

9. The eye drop formulation as claimed in claim 7, wherein the polyvinylpyrrolidone is present in a concentration of 0.5 to 5.0 w/v%.

10. The eye drop formulation as claimed in claim 7, wherein the hydroxypropyl methylcellulose has a weight average molecular weight of 10,000 to 1,500,000.

11. The eye drop formulation as claimed in claim 7, wherein the hydroxypropyl methylcellulose is present in a concentration of 0.1 to 0.4 w/v%.

12. The eye drop formulation as claimed in claim 7, further comprising polyethylene glycol and polysorbate as a solubilizing agent.

13. The eye drop formulation as claimed in claim 12, wherein the polyethylene glycol has a weight average molecular weight of 380 to 420.

14. The eye drop formulation as claimed in claim 12, wherein the polyethylene glycol is present in a concentration of 2.0 to 5.0 w/v%.

15. The eye drop formulation as claimed in claim 12, wherein the polysorbate is polysorbate 20, polysorbate 40, polysorbate 60, or polysorbate 80.

16. The eye drop formulation as claimed in claim 12, wherein the polysorbate is present in a concentration of 0.05 to 0.15 w/v%.

17. The eye drop formulation as claimed in claim 1, which comprises:
0.1 to 1.0 w/v% of 8-oxo-2'-deoxyguanosine or a pharmaceutically acceptable salt thereof;
0.5 to 2.0 w/v% of a buffering agent;
0.01 to 0.3 w/v% of a chelating agent;
0.5 to 5.0 w/v% of polyvinylpyrrolidone;
0.1 to 0.4 w/v% of hydroxypropyl methylcellulose;
2.0 to 5.0 w/v% of polyethylene glycol; and
0.05 to 0.15 w/v% of polysorbate,
in an aqueous medium.

18. The eye drop formulation as claimed in claim 1, which comprises:
0.25 w/v% of 8-oxo-2'-deoxyguanosine;
1.0 w/v% of tromethamine;
0.1 w/v% of sodium ethylenediaminetetraacetate;
2.0 w/v% of polyvinylpyrrolidone;
0.4 w/v% of hydroxypropyl methylcellulose;
2.0 w/v% of polyethylene glycol; and
0.15 w/v% of polysorbate,
in an aqueous medium.
